# EUROPEAN PATENT SPECIFICATION

(11) **EP 3 482 792 B1**
(45) Date of publication and mention of the grant of the patent: **13.01.2021**
(21) Application number: 18200001.8
(22) Date of filing: 12.10.2018
(51) Int. Cl.: A61M 35/00

(54) **A DISINFECTANT APPLICATOR**
DESINFEKTIONSMITTELAPPLIKATOR
APPLICATEUR DE DÉSINFECTANT

(30) Priority: 10.11.2017 TR 201717714
(43) Date of publication of application: 15.05.2019
(73) Proprietor: ALTERA TIBBI MALZEME SANAYI VE TICARET ANONIM SIRKETI, Tire/Izmir (TR); Sevincli, Atilla, Izmir (TR)
(72) Inventor: SEVINCLI, ATILLA, Izmir (TR)
(74) Representative: Kaya, Erdem

(56) References cited:
- US-A1- 2007 196 159
- US-A1- 2011 066 121

## Description

### TECHNICAL FIELD

The invention is related to a disinfectant applicator that enables application of the optimum amount of disinfectant product to the operation area of pre-operative patients.

### PRIOR ART

While disinfectant is being poured onto the patient directly from the bottle, the disinfectant leaves color splashes to the surrounding or to parts of the patient which do not require contact with the disinfectant. When it is desired to apply the disinfectant to the patient by first pouring onto a gauze bandage, the disinfectant splashes to the surrounding area while being poured onto the gauze bandage. In both application ways, more than the needed product is used and wasted and this brings an additional cost to the hospital. The problems experienced with the applications performed by the applicators available in markets are that these applicators contain a standard amount of disinfectant and thus they cannot be used for another patient in case there is still disinfectant left inside since these are only for single use and the remaining disinfectant is wasted or that a plurality of products must be used since the product contains disinfectant less than required and thus new products must be opened continuously or again there disinfectant remains in the last product that has been opened. Since these products are considered as biocidal products due to the disinfectant they contain, in Turkey it is mandatory to obtain a license. They cannot be sold as medical devices.

In the current state of the art, before the surgery, in order to disinfect the parts of the patient to be cut due to a surgery, povidone iodine or other biocidal products that perform disinfection which are generally sold in 1 L volume bottles are used. In order to apply (wipe) these products to the related area, the disinfectant liquid is poured onto a gauze bandage and the patient is wiped with a gauze bandage or the disinfectant liquid is poured onto the patient and applied by wiping with the gauze bandage. While this method is being also used, there are some products to facilitate said disinfection procedure. Among them, the leading ones are ChloraPrep from BD and DuraPrep from 3M companies. These products are usually produced in 26 ml, 6 ml, 3 ml and 1 ml packages. ChloraPrep and some DuraPrep types enable pouring the liquid onto the sponge at the tip by breaking the structure inside the applicator containing the disinfectant via a latch. Some DuraPrep types enable the liquid to reach the sponge at the tip by pumping from the back of the applicator. Related body part of the patient is disinfected by wiping the sponges in the products directly onto the patient

US2011/066121 and US2007196159 disclose applicators which have lumens. US2011/066121 discloses applicators which have breakable membrane and user has to broke or degenerate this membrane with a part of apparatus or applying lateral force to reservoir. US2007196159 discloses applicator which has deformable receiver section and via the applicator through manual deformation of the foldable bag like receiver section, flow connection occurs.

### BRIEF DESCRIPTION OF THE INVENTION

The present invention is related to a disinfectant application applicator aimed for eliminating the above-mentioned disadvantages and to bring new advantages to the related technical field.

The invention consists of a special and ergonomically designed bottle that contains disinfectant and an ergonomically designed disposable (one-time use) apparatus that is completely compatible with the disinfectant bottle which enables application of this disinfectant on the patient. Due to its soft sponge containing apparatus, the disinfectant is easily applied to the patient as desired, it is not required to pour the disinfectant on the patient or on the gauze bandage and thus waste and cost increase is avoided. Thanks to its removable/replaceable apparatus, after a desired quantity of disinfectant is used, cap of the bottle can be closed and the bottle can be used for another patient with a newly opened apparatus. Thus, economical use can be provided.

In order to achieve all the objects that are mentioned above and that may become clear from the detailed description provided below, the present invention is related to the application of an optimum quantity of disinfectant on operation area of pre-operative patients. Said disinfectant applicator comprises a disposable apparatus which enables ergonomically handling of the applicator and on which a disinfectant containing bottle can be installed and removed, a cylindrical plug having circular holes which is positioned inside the apparatus on the liquid side, a preferably circular sponge that enables application of the liquid passing through the plug onto the surface and that is positioned at the tip of the apparatus and liquid transfer pipes that enable liquid transfer between the bottle and the sponge.

A preferred embodiment of the invention is characterized by comprising a threaded structure (5) inside the disposable apparatus (2) that enables installation of the bottle (1) by rotating.

### BRIEF DESCRIPTION OF THE FlGURES

Figure 1 shows the vertical cross-sectional view of the invention.

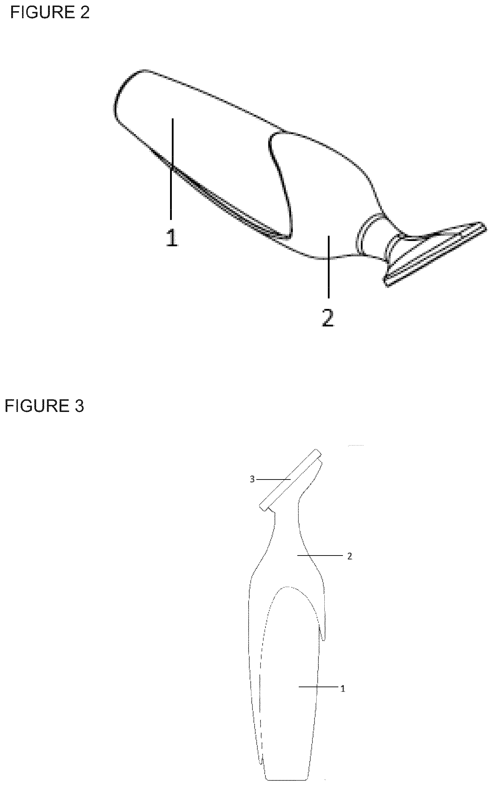

Figure 2 shows the isometric external view of the invention.
Figure 3 shows the side external view of the invention.

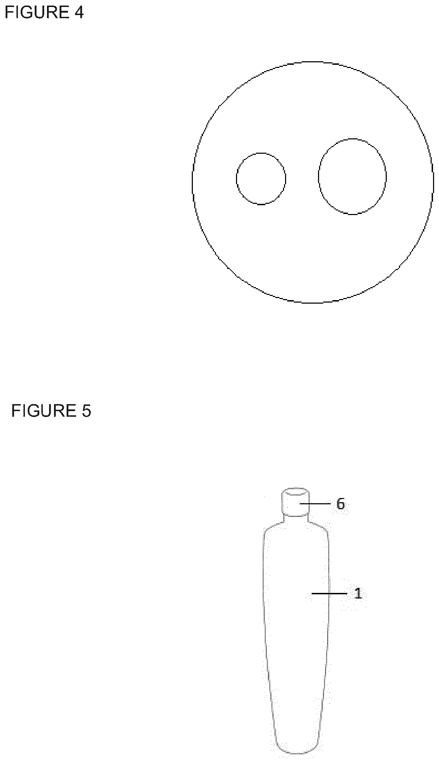

Figure 4 shows the cross-sectional view of the plug contained in the invention.
Figure 5 shows the disinfectant bottle.

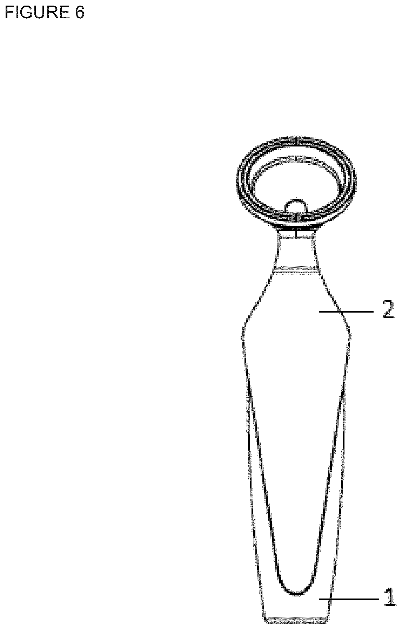

Figure 6 shows the bottom view of the invention.

### REFERENCE NUMBERS

1. Bottle
2. Disposable Apparatus
3. Sponge
4. Plug
5. Threaded Structure
6. Cap
7. Pipe

### DETAI LED DESCRIPTION OF THE INVENTION

In this detailed description, the novelty of the invention is illustrated by non-limiting examples in order to provide a better understanding of the subject matter. The disinfectant application applicator and its constituents are described.

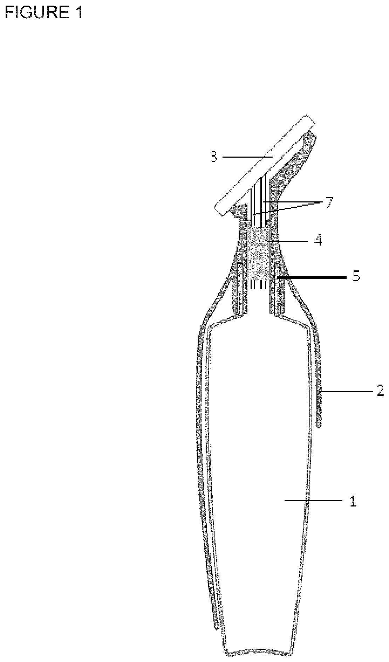

The invention is related to an applicator that enables application of an optimum quantity of disinfectant to the operation area at pre-operative patients. Basically, it comprises a disposable apparatus (2) which enables ergonomically handling of the applicator and on which a disinfectant containing bottle (1) can be installed and removed, a cylindrical elastomeric plug (4) having circular holes which is positioned inside the apparatus (2) on the liquid output side of the bottle (1), a preferably circular sponge (3) that enables application of the liquid passing through the plug (4) onto the surface and that is positioned at the tip of the apparatus and at least one liquid transfer pipe (7) that enables liquid transfer between the bottle (1) and the sponge (3). The bottle (1) and the apparatus (2) are independent parts that can be assembled together. The bottle (1) is produced full of a registered biocidal product or disinfectant preferred by the end user and it is delivered to the end user with its cap (6) closed and in a sterile manner. The disposable apparatus (2) is assembled with the sponge (3) and the plug as shown in Figure 1 and it is produced in a sterile manner. The disposable apparatus (2) is delivered to the end user with the filled disinfectant applicator bottles (1) depending on the needs of the end user. Prior to use of disinfectant, the bottle (1) is opened by turning the bottle cap (6). The apparatus (2) is installed on the bottle (1) by turning clockwise by using the bottle-apparatus threaded installation interface (5) specially designed in accordance with the cap threads of the bottle. The bottle (1) connected to the apparatus (2) is turned upside down such that the sponge (3) contacts the surface to be disinfected. Thus, the disinfectant liquid progresses through the liquid transfer pipes (7), that are produced from soft or hard elastomeric materials and that is fixed by the plug (4), and reaches the sponge (3). By establishing an uninterrupted link between the liquid and the sponge (3), the liquid flows such that the liquid concentration, that is related to the application speed of the disinfectant liquid to the patient, stays constant on the sponge (3) until the liquid inside the bottle (1) is over. The thinner the liquid transfer pipes (7), a more accurate control of the liquid flow is possible. When a faster liquid flow is desired, the applicator bottle (1) can be squeezed to apply pressure. When the pressure is removed, the bottle returns to its initial shape and the excess liquid on the sponge (3) is retracted by vacuum and the liquid returns back to its normal flow rate. The liquid transfer pipes (7) also help the disinfectant liquid to disperse around different parts of the sponge (3). The plug (4) and the liquid transfer pipes (7) together stabilize and optimize the liquid flow and the sponge (3) enables gentle application of the disinfectant to the skin as required. Use of disinfectant applicator is very easy due to its special easy handled ergonomic design. After a required quantity of disinfectant is used, independent from the amount of the disinfectant inside the bottle (1), the disinfectant applicator is positioned such that the sponge (3) faces upwards and the bottle (1) and the apparatus (2) are separated from each other by turning counterclockwise. If there is any disinfectant left inside the bottle (1), the bottle can be stored for later use after closing the cap(6). However, the apparatus (2) is disposable and it should be disposed after use according to hospital regulations.

The disinfectant application applicator is in two pieces. The bottle containing the disinfectant liquid (1) and the disposable apparatus (2) enabling the applicator are separately packaged. The bottle (1) and the apparatus (2) are designed compatible with the hand ergonomics. When desired, the apparatus (2) can be used with the bottle (1) through the bottle-apparatus threaded connection point (5) which makes them compatible with each other. After use, they can be separated and the bottle (1) can be stored with the disinfectant therein and can be used by installing another apparatus (2) for another person. In the products available in foreign markets, the disinfectant liquid is inside the monoblock applicator and the section containing the liquid is broken by a latch or by applying pressure on the special part at the back of the applicator or by turning the head of the applicator or the sleeve preventing the liquid from flowing is torn. The disinfectant disperse over the sponge on the product and used once. It is thrown away regardless of if there is any disinfectant left inside after use. The disinfectant is run by squeezing depending on the squeezing pressure or by gravity. There is no physical mechanism other than thickening the sponge or installing additional sponge for flow rate control. In our invention, the flow of disinfectant takes place from the bottle (1) through the liquid transfer pipe (7) and to plug (4) inside the apparatus (2) and then towards the sponge (3). The liquid transfer pipes (7) positioned on the plug (4), of which the capillarity is adjusted according to the sponge (3) diameter, enable that the flow of disinfectant is not too much to cause dripping from the sponge (3) or too little to require waiting for too long. Thanks to these pipes, an uninterrupted link is established between the liquid and the sponge (3) so that the flow runs until the liquid inside the bottle (1) is over such that the liquid concentration and speed of flow on the sponge (3) is kept constant that is the application pace of the disinfectant liquid to the patient. When an increased flow rate of the liquid is desired, the applicator bottle (1) can be squeezed to apply pressure. When squeezing pressure is removed, the bottle (1) returns to its initial shape and the excess liquid on the sponge (3) is retracted by vacuum and the liquid returns to its regular flow rate. Our invention is used to disinfect the skin positioned on the region to be operated in surgeries.

## Claims

1. A disinfectant applicator that enables application of an optimum quantity of disinfectant to operation area in pre-operative patients, **characterized by** comprising;
a disposable apparatus (2) that enables ergonomic handling of the applicator and on which a disinfectant containing bottle (1) can be installed and removed,
a cylindrical elastomeric plug (4) having circular holes which is positioned inside the apparatus (2) on the liquid output side of the bottle (1),
the sponge (3) that enables application of the liquid passing through the plug (4) onto the surface and that is positioned at the tip of the apparatus and
two transfer pipes (7) that enables liquid transfer between the bottle (1) and the sponge (3).

2. An applicator according to Claim 1, **characterized by** comprising a threaded structure (5) inside the disposable apparatus (2) which enables installation of the bottle (2) by turning.

## Patentansprüche

1. Applikator für Desinfektionsmittel zum Auftragen eines Desinfektionsmittels in optimaler Menge auf einen zu operierenden Körperbereich eines präoperativen Patienten, **gekennzeichnet durch**:
eine Einwegvorrichtung (2), die eine ergonomische Handhabung des Applikators ermöglicht und auf der eine Desinfektionsmittel enthaltende Flasche (1) angebracht und entfernt werden kann;
einen zylindrischen Elastomerstopfen (4) mit kreisförmigen Löchern, der innerhalb der Vorrichtung (2) auf der Flüssigkeitsausgangsseite der Flasche (1) angeordnet ist;
einen Schwamm (3), der zum Auftragen der durch den Stopfen (4) austretenden Flüssigkeit auf eine Oberfläche dient und stirnseitig an der Vorrichtung angebracht ist, und
zwei Überführungsrohre (7), die die Übertragung von Flüssigkeit zwischen der Flasche (1) und dem Schwamm (3) ermöglichen.

2. Applikator nach Anspruch 1, **gekennzeichnet durch** eine innerhalb der Einwegvorrichtung (2) angeordnete Gewindestruktur (5), die das Anbringen der Flasche (2) durch eine Drehbewegung ermöglicht.

## Revendications

1. Un applicateur de désinfectant qui permet l'application d'une quantité optimale de désinfectant à la zone d'opération chez des patients préopératoires, **caractérisé en ce qu'**il comprend;
un appareil jetable (2) qui permet une manipulation ergonomique de l'applicateur et sur lequel une bouteille (1) contenant un désinfectant peut être installé et retiré,
un bouchon (4) élastomère cylindrique ayant des trous circulaires qui est positionné à l'intérieur de l'appareil (2) du côté de sortie de liquide de la bouteille (1),
l'éponge (3) qui permet l'application du liquide qui passe à travers le bouchon (4) sur la surface et qui est positionnée à l'extrémité de l'appareil et deux tuyaux de transfert (7) qui permettent le transfert de liquide entre la bouteille (1) et l'éponge (3).

2. Un applicateur selon la Revendication 1, **caractérisé en ce qu'**il comprend une structure filetée (5) à l'intérieur de l'appareil jetable (2) qui permet l'installation de la bouteille (2) en tournant.
